(19) **Europäisches Patentamt**

**European Patent Office**

**Office européen des brevets**

(11) Publication number: **0 215 533 B1**

(12) **EUROPEAN PATENT SPECIFICATION**

(45) Date of publication of patent specification: **15.12.93**

(51) Int. Cl.⁵: **C12N 15/10**, C12P 19/34

(21) Application number: **86300075.8**

(22) Date of filing: **07.01.86**

The file contains technical information submitted after the application was filed and not included in this specification

(54) **A method of extracting nucleic acids from cells.**

(30) Priority: **18.01.85 US 692958**

(43) Date of publication of application:
**25.03.87 Bulletin 87/13**

(45) Publication of the grant of the patent:
**15.12.93 Bulletin 93/50**

(84) Designated Contracting States:
**BE CH DE FR GB IT LI SE**

(56) References cited:
**EP-A- 0 008 189     EP-A- 0 145 356**
**CH-A- 542 924       DD-A- 230 560**
**FR-A- 1 295 038      GB-A- 1 157 361**
**GB-A- 2 143 238      US-A- 3 389 133**
**US-A- 4 338 285**

**MANIATIS et al., "Molecular Cloning, A laboratory manual", CSH symposia, 1982.**

**BIOCHEMISTRY, vol. 16, no. 24, 1977, pp. 5329-5341, US; D.E. KOHNE et al. "Room temperature method for increasing the rate of DNA reassociation by many thousandfold: The phenol emulsion reassociation technique"**

**PATENT ABSTRACTS OF JAPAN, vol. 5, no. 168, 27th October 1981 (C-77) (840)**

(73) Proprietor: **APPLIED BIOSYSTEMS, INC.**
**850 Lincoln Centre Drive**
**Foster City California 94404(US)**

(72) Inventor: **Cathcart, Guy Richard**
**525 Colusa Avenue, Berkeley**
**California 94707(US)**
Inventor: **Grossman, Paul David**
**1131 Compass Lane**
**No. 303, Foster City California 94404(US)**
Inventor: **Whiteley, Norman**
**151 Highland Avenue**
**San Carlos California 94070(US)**

(74) Representative: **West, Alan Harry et al**
**R.G.C. Jenkins & Co.**
**26 Caxton Street**
**London SW1H 0RJ (GB)**

**Description**

This invention relates to the isolation and purification of nucleic acids from cells, and particularly to apparatus for automatically achieving such isolation.

One of the first steps in the in vitro manipulation of nucleic acids involves their isolation, for example relatively pure samples of genomic DNA are required in order to perform tests for genetic disease and recombinant technology requires isolation of both the vector DNA and the DNA to be cloned.

As a general rule, DNA does not exist as a free molecule in a cell, but instead exists as a complex association of DNA, RNA and proteins. This is a consequence of the role of DNA as the genetic blueprint of the cell. In order to express genetic information, the DNA is used as a template for the production of messenger RNA, which is translated by the ribosome into protein. Proteins directly involved in the process of gene expression, such as RNA polymerase and regulatory proteins, interact with DNA in vivo to form nucleo-protein complexes. DNA polymerase, DNA ligase, various unwinding and supercoiling enzymes, recombination and repair enzymes, and those proteins involved in the initiation or maintenance of DNA replication are also associated with DNA in vivo and hence complicate the isolation of pure DNA. Because of this complex association of DNA with these other proteins and nucleic acids, the purification (isolation) approach for obtaining DNA can generally be thought of as a three step process: (1) releasing soluble, high molecular weight DNA from disrupted cell wall and membranes; (2) dissociating DNA-protein complexes by protein denaturation or proteolysis; and (3) separating DNA from the other macromolecules.

Within this process, DNA of bacterial origin (prokaryotic DNA) is typically purified by different methods, depending on whether the DNA is chromosomal or extrachromosomal, such as plasmids or bacteriophage. In the purification of chromosomal DNA, the bacterial cell wall is generally weakened by freeze-thawing or by treatment with the enzyme lysozyme and the chelating agent ethylenediaminetetraacetic acid (EDTA). Cell lysis is accomplished by the addition of a detergent such as sodium dodecyl sulfate (SDS) in a buffered saline solution. Following lysis, the solution is treated with pancreatic ribonuclease to hydrolyze RNA and protease to degrade proteins. Residual proteins and oligopeptides are extracted with an organic solvent, such as phenol or an equal mixture of phenol and chloroform. Most of the protein will denature and enter the organic phase or precipitate at the interface of the organic and aqueous phases, this phase separation being accomplished by means of centrifugation. The clear, viscous aqueous phase containing the DNA is then removed. With the addition of alcohol, the DNA precipitates out of the aqueous phase as a white fibrous material and can be spooled onto a glass rod. Precipitation from alcohol serves to concentrate the high molecular weight DNA while removing the small oligonucleotides of DNA and RNA, detergent, and the organic solvent used in the removal of proteins. Residual detergent and salts can be removed by dialysis of the resuspended DNA solution against the desired buffer. In some instances, it may be desirable to further purify the DNA by centrifugation on isopycnic cesium chloride gradients, or by hydroxylapatite chromatography. In the above process for chromosomal DNA, typical protocols often require at least two days for the DNA extraction and purification process. (See Recombinant Techniques by Raymond L. Rodrigues, and Robert C. Tact, 1983, p. 162).

During the purification of extrachromosomal elements of prokaryotic DNA, including plasmids and bacteriophage, it is desirable to minimize the amount of chromosomal DNA contaminating the preparation. With bacteriophage, this is often accomplished by first purifying the phage particles from the infected bacteria, then treating the purified phage particles with protease and/or phenol to release the bacteriophage DNA. Further purification of the DNA is accomplished by means similar to those described for chromosomal DNA. Due to its size, however, precipitated bacteriophage and plasmid DNA cannot be spooled out on a glass rod and is therefore generally recovered by centrifugation. Again, three days is not atypical for the entire isolation and purification process.

For eukaryotic cells, isolation and purification of total cellular DNA is often achieved by a modification of the detergent lysis procedure described above for bacteria. The key difference is that typically cell lysis and digestion of cellular proteins are accomplished using proteinase K in the presence of the detergent. (See M. Gross-Bellard, P. Oudet, and P. Chambon, Eur. J. Biochem., 36(1973) 32-38; N. Blin, and D.W. Stafford, Nuc. Acid. Res., 3(1976) 230-2308; and D.J. Law, P.M. Frossard and D.L. Rucknagel, Gene, 28 (1984) 153-158). The proteinase K is then removed by extraction of the lysate with phenol or a phenol/chloroform mixture. Typically, in the mixing process as for the extraction of bacterial DNA, the lysate/phenol or lysate/phenol-chloroform forms an emulsion, the aqueous and organic phases of which are separated by centrifugation. The upper, or aqueous phase containing the DNA is then poured off or removed using a pipette, and this essentially protein-free lysate is dialyzed to remove small molecular weight cellular contaminants and residual phenol.

In the above approaches, a major limitation on the extraction which critically limits the ability to automate the process, is the need for centrifugation to separate the aqueous and organic phases during the phenol extraction. Often several extractions are required to achieve the desired purity, each one requiring centrifugation. Largely due to these various centrifugations, the work is performed manually and is therefore expensive. Also these configurations make automating of the extraction process difficult and expensive.

Accordingly, the present invention provides a method of extracting nucleic acid from cells which includes the steps of

(a) creating a lysate by treating said cells with proteinase K in the presence of a lysis buffer;

(b) mixing said lysate with a phenol-based solvent system, thereby creating an emulsion; and

(c) separating the phases into an aqueous phase containing said nucleic acids and an organic phase containing phenol and denatured proteins characterised by

(i) heating said emulsion to a temperature greater than 45°C prior to separation to cause phase separation; and

(ii) removing said organic phase without centrifugation.

Furthermore, the present invention provides an apparatus for extracting nucleic acids from cells, the apparatus comprising:

extraction vessel means for holding said cells;

delivery means for delivering reagents to said extraction vessel means, and for removing fluids from said extraction vessel means;

heater means for heating said extraction vessel means;

temperature measuring means for measuring the temperature of said extraction vessel means;

motor means for oscillating said extraction vessel means to mix said cells and said reagents;

computer means for controlling said heater means, said motor means and said delivery means, for controlling volume of flow of said reagents into said extraction vessel means, for monitoring the temperature measured by said temperature measuring means, for monitoring flow of fluids from said extraction vessel means, for timing the heating of said extraction vessels by said heating means, and for timing the mixing by said motor means, said computer means operating according to a preselected instruction set to cause extraction of said nucleic acids from said cells without centrifugation.

In accordance with a preferred embodiment of the invention, an automated apparatus is provided which implements a new method of extracting and purifying nucleic acids from cells without the use of centrifugation. In the method, a lysate is created by treating the cells with proteinase K in the presence of a lysis buffer having a high concentration of a salt. The lysate is mixed with a phenol-based solvent system, thereby creating an emulsion. The emulsion is heated to a temperature of at least 45°C to promote phase separation. For optimum results, a temperature of about 55°C is preferred. The rate of phase separation is also enhanced by increasing the surface surface area of the emulsion. Once the phase separation is complete, the lower organic phase is removed and the upper aqueous phase is repeatedly extracted with the phenol-based solvent a preselected number of times, and is finally extracted using chloroform. The remaining aqueous phase is then dialyzed to further purify and concentrate the nucleic acid solution.

The apparatus for implementing this method includes at least one extraction vessel for holding the sample cells and a delivery system for delivering reagents to the extraction vessel and for removing fluids from the extraction vessel. A heating system is provided for maintaining the desired temperature of the extraction vessel during the phase separation process, and a motor is used for gently oscillating the extraction vessel to obtain mixing of the fluids contained therein. The motor also rotates the extraction vessel about a horizontal axis to achieve an increase in surface area of the emulsion resulting from treatment of the lysate with the phenol-based solvent system. The combination of the high salt concentration together with heating and increasing the surface area of the emulsion results in phase separation in 2 to 8 minutes, and thus totally eliminates the need for centrifugation.

A computer system is used for controlling the heating system, the motor, and the delivery system, for timing the flow of the reagents into the reaction vessel to control volume, for monitoring the temperature in the reaction vessel, and for monitoring the flow of the organic phase out of the extraction vessel. The computer also serves as the master timer, timing the mixing by the motor and the wait time during phase separation, and operates according to a preselected instruction set based on the above method. A pressurized dialysis system is also included in the apparatus and is coupled to the extraction vessel by the delivery system. The dialysis system includes a pump for recirculating dialysate from a bath vessel through a spectrophotometer and a filter system back into the bath vessel in order to reuse the dialysate and to avoid excessive replenishment.

3

Brief Description of the Drawings

Fig. 1 shows a schematic representation of a fluid delivery system according to the invention.

Figs. 2A and 2B show two views of a chamber/rocker system according to the invention.

Fig. 3 shows a pressurized dialysis system according to the invention.

Fig. 4 shows a schematic representation of a computer system according to the invention.

Fig. 5 illustrates a preferred design for an extraction vessel and its attachment in the apparatus.

Fig. 6 illustrates the details of a suspension system for dialysis bags in the dialysis system.

Detailed Description of the Invention

Definitions

For the purpose of the description of the invention, the following definitions will apply:

An "emulsion" is a mixture of two immiscible liquids which are kept in suspension, one within the other. In the context of the extraction of nucleic acids from cells, after cell lysis and mixing of the lysate with a phenol-based solvent system, an emulsion is formed, the constituent fluids of which are an aqueous phase containing the nucleic acids, and an organic phase containing the phenol, denatured proteins, and lipids. In addition to nucleic acids, the aqueous phase also typically contains impurities which in many situations must be removed before further in vitro manipulations can be performed. These impurities include trace amounts of the phenol-based solvent system, many small molecular weight cellular constituents such as carbohydrates, amino acids, and smaller nucleic acids such as nucleosides and nucleotides (usually referred to as the cell sap).

"Dialysis" is a separation process that depends on the differential transport of solutes of different sizes across a porous barrier separating two liquids where the driving force is a concentration gradient only. In the extraction of nucleic acids, dialysis is often used to remove the impurities in the aqueous phase of the emulsion.

"Restriction" is the selective cleaving or endonucleitic cleaving of DNA at unique base sequence sites. Generally, restrictability is considered a strigent test for DNA purity.

Method

The approach of the invention relies on automation of a new method to achieve separation of the organic (phenol) and aqueous (DNA and/or RNA) phases during a nucleic acid extraction without the use of centrifugation. The protocol used is adapted to extraction of nucleic acids from mammalian cells and particularly to high molecular weight DNA (about $10^8$ daltons) from tissues such as peripheral blood lymphocytes, liver, and amniocytes, although it can also be used for other eukaryotic cells if the tissue is properly prepared before the extraction is performed. The protocol is as follows:

Step 1. The tissue from which the nucleic acid is to be extracted is digested with the enzyme proteinase K in the presence of a lysis buffer which has a high concentration of a salt, the salt increasing the ionic strength. The preferred lysis buffer is the mixture 8M urea; 1M NaCl; 1% SDS, 50mM Tris-HCl, and 10mM EDTA, pH 8.0. The digestion is typically performed at about 55°C for 3 to 6 hours, depending on the nature of cells to be digested. The 8M urea concentration corresponds approximately to saturation and so represents an upper limit. Somewhat lower urea concentrations can be used, although 8M is preferred for best efficiency. A lower limit appears to be about 4M urea. Also, other chaotropic agents, e.g., guanadine hydrochloride, may be substituted for urea. The concentration of the detergent SDS can also be varied, typically from as low as 0.5% to as much as 2%, but a concentration of about 1% appears to be more than adequate for most types of mammalian cells. Other detergents such as Triton X-100™, Nonedit™, and lauroyolsarcosine may also be used. Similarly, the high salt concentration can be varied from 0.5M, which considerably slows down phase separation in Step 3 below, to as high as 2M, which does not seem to appreciably increase the rate of phase separation over that obtained with the preferred 1M NaCl solution. Other salts, e.g., KCl, may also be used, the preferred concentration depending on the ionic strength of the salt used. Also, chelating agents other than EDTA may be used, for example 8-hydroxyquinoline, and in some instances, the chelating agent may be omitted. The Tris-HCl serves as a buffer.

Step 2. The lysate resulting from Step 1 is gently mixed at room temperature for about 20 minutes with an equal volume of a phenol-based solvent system, preferrably phenol/chloroform/isoamyl alcohol at ratios of about 50:48:2, the phenol for denaturing and extracting the proteins, the chloroform to increase the hydrophobicity, and to ensure that the DNA remains in an aqueous phase (see Step 3), and the isoamyl alcohol to serve primarily as an antisurfactant (antifoaming agent). An emulsion is then formed as a result of the mixing. Variations on this organic solvent system may be used, such as replacing the phenol/chloroform/isoamyl alcohol system with a phenol saturated with Tris-HCl buffer, pH 8.0, but the phenol/chloroform/isoamyl alcohol system is preferred because of its efficiency in effecting the desired phase separation in Step 3, and because the DNA does not get lost in the interface between the organic and aqueous phases, as can happen with the system using phenol saturated with buffer. Similarly, the precise volumetric ratios (i.e., 50:48:2) of this preferred phenol based solvent system can be varied, but too low of a concentration of chloroform often permits the DNA to enter the organic phase rather than remain in the aqueous phase. As a general rule, a ratio of phenol to chloroform of about 1:1 seems to provide optimum performance. Similarly, too low of a concentration of the antisurfactant can result in foaming which can clog the tubes.

Step 3. Phase separation of the emulsion resulting from Step 2 is then accomplished by increasing the surface area of the emulsion while heating to a temperature preferably between 45°C and 55°C, more preferably to about 55°C, that latter temperature being close to the boiling point of chloroform. Increasing the surface area is typically done by positioning the reaction vessel containing the emulsion on its side which increases the cross-sectional area of the interface between the lysate and organic phase and decreases the depth of the two phases. The combination of the high concentration of salt, the large interface area, and the high temperature during this step causes the emulsion to separate into a two-phase system in 2 to 8 minutes, thus totally eliminating the need for centrifugation.

Step 4. The reaction vessel is slowly returned to its normal upright position to maintain the phase separation.

Step 5. The lower phenol phase is withdrawn and educted to waste.

Step 6. The extraction procedure, Steps 2 through 5 above are repeated until the upper phase containing the nucleic acids is clear. Typically only one additional extraction is required for the isolation of nucleic acids from lymphocytes, whereas for some other cell types, for example liver, as many as three additional extractions may be required. Typically, the final extraction is performed with chloroform alone which removes most of the residual phenol, rather than with the phenol/chloroform/isoamyl alcohol mixture.

Step 7. The aqueous phase remaining after Step 6 is removed.

Step 8. The solution removed in Step 7 is dialyzed, typically under pressure, to concentrate the nucleic acids and to remove residual organic molecules.

Step 9. As an optional step, after Step 8, the aqueous phase can be treated with either RNase or DNase, and the extraction Steps 2 through 8 repeated to leave only DNA or RNA, respectively, in the aqueous phase.

Step 10. Collect the purified sample.

Apparatus

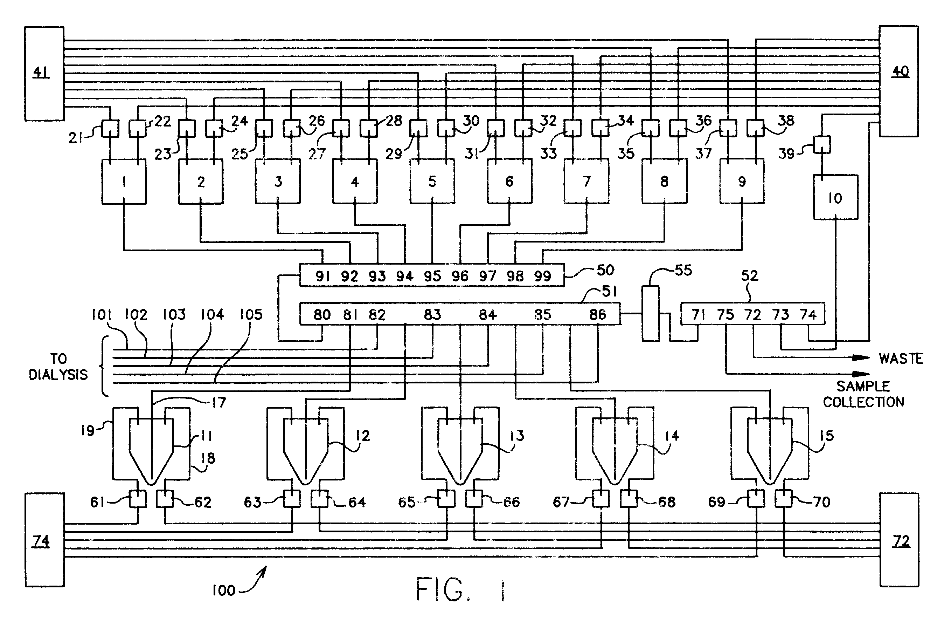

In accordance with a preferred embodiment of the invention, an apparatus for isolating and purifying nucleic acids from cells is illustrated in Figs. 1, 2A and 2B, 3, and 4, which show respectively, a fluid delivery system 100 for routing the various reagents and gases throughout the system; a chamber/rocker system 200 for controlling the environment and the physical orientation of extraction vessels during the extraction process; a dialysis system 300; and a computer system 400 for effecting automatic control.

The fluid delivery system 100 illustrated in Fig. 1 includes a series of reagent vessels, 1 through 9, and a series of pairs of electrically operated gas valves 21 through 38, with one pair valves for each reagent vessel. Each gas valve of the series 21 through 38 is connected either to a pressure manifold 40 or to a vent manifold 41, and to a particular reagent vessel in the series 1 through 9, in order to control the pressure in that reagent vessel. Such reagent vessels are typically constructed of glass or polyethylene, depending on the reagent contained therein. For the particular protocol used in the extraction process described above, the reagents include the enzyme proteinase K; the lysis buffer made up of 8M urea, 1M NaCl, 1% SDS, 50mM Tris-HCl, and 10mM EDTA, pH 8.0; the mixture of phenol/chloroform/isoamyl alcohol (50:48:2); chloroform; and RNase and/or DNase. For other protocols, such as for extractions from bacteria and yeasts, other reagents which might be used would include for example, lysozyme, SDS, ethanol, Tris-HCl, EDTA, various saline solutions, and other buffers.

Each of the reagent vessels is coupled to an electrically operated valve block 50, which is an assembly of zero dead volume valves similar to those described in U.S. Patent 4,008,736, issued February 22, 1977, entitled VALVE ARRANGEMENT FOR DISTRIBUTING FLUIDS, by Wittman-Liebold, et al., as are all other valve blocks in the system. An example of such an electrically operated valve block is manufactured by Applied Biosystems, Inc., part number 600656. Another example is described in copending application "Improved Apparatus and Method for the Sequential Performance of Chemical Processes," filed November 10, 1982, Serial Number 440,571,k by L. E. Hood, et al., assigned to the same assignee. Fluid flow from the various reagent vessels is controlled by opening the appropriate gas valve and closing the appropriate vent valve to increase the pressure in the desired reagent vessel and opening the appropriate valve in valve block 50. A valve block 51 which is coupled to valve block 50 then directs the flow to a particular extraction vessel, one of vessels 11 through 15. Flow into these extraction vessels is controlled via a series of pairs of electrically operated gas valves 61 through 70, each of which is coupled to either a gas manifold 72 or to a vent manifold 74. Once the extraction in the extraction vessels is complete and organic and aqueous phases have been separated, the organic phase (which is on the bottom in the extraction vessels) is removed by increasing the pressure in the desired extraction vessel, and opening the corresponding valve in valve block 51, thus forcing the fluid out of the vessel through an educting tube, such as tube 17 on extraction vessel 11. Valve block 51 directs the flow through a conductivity detector 55 to another valve block 52 and to waste. A large increase in conductivity is seen when the organic phase has been educted, since the aqueous phase has a high conductivity due to its high salt content. This provides the signal necessary to the computer system to indicate that the phase interface has been detected and to stop any further removal of fluid from the extraction vessel. Once that signal has been received, the waste valve in valve block 52 is closed and either the appropriate dialysis ports of valve block 51, i.e., one or more of ports 82 through 86, are opened to educt the aqueous phase to the dialysis system 300 or the dialysis ports are closed forcing the fluid of the aqueous phase remaining in the fluid delivery lines back into the appropriate reaction vessel in preparation for another phenol/chloroform/isoamyl alcohol extraction. A gas valve 39 is used to force a buffer such as Tris-HCl from an extraction vessel 10 into block valve 52 for backflushing.

Tubing such as tube 16 in the above apparatus for connecting the various vessels and valves is typically constructed of Teflon™. Each tube for transferring liquids in the system has a roughly calibrated flow resistance and operates at a fixed known pressure during transfers due to the pressure manifolds. Hence, the length of time required for a transfer corresponds directly to the volume of material transferred and is controlled by the computer system. Gas flows throughout the system are also controlled by the computer system, the gas valves 21 through 39 and 61 through 70 typically being solenoid operated. An example of such valves includes fluorocarbon valves made by Angar, Inc. An example of an appropriate conductivity detector 55 is a Wescon Instruments Model 219-200 conductivity flow cell coupled to a Model 212-100 conductivity meter.

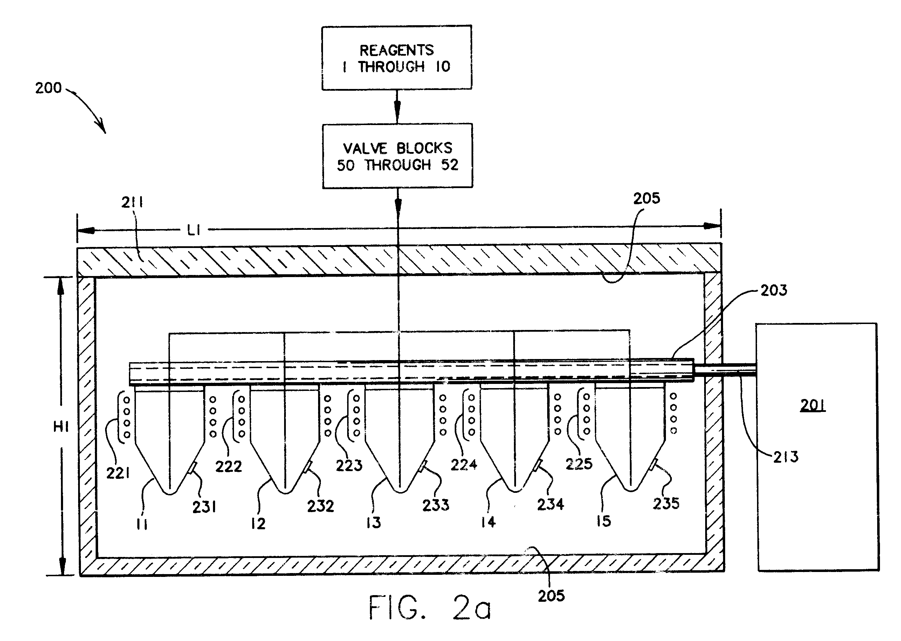

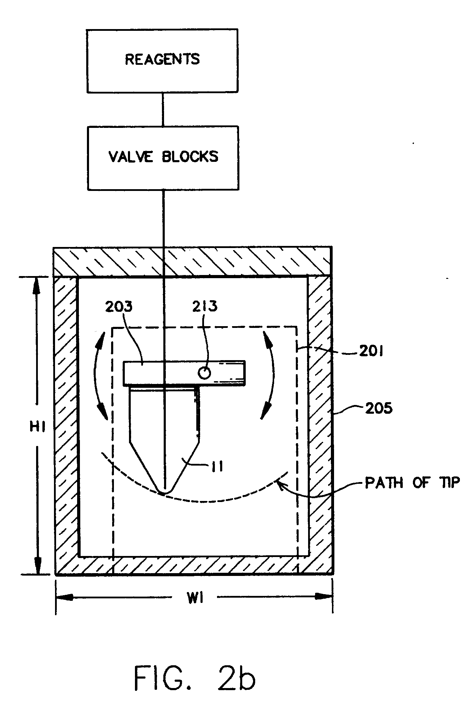

Shown in Figs. 2A and 2B is the chamber/rocker system 200 which includes a motor 201, a rocker arm 203, and an insulated chamber 205 containing band heaters 221 through 225 (one for each extraction vessel), and corresponding thermisters 231 through 235, and the extraction vessels 11 through 15. In a typical implementation, chamber 205 is a rectangular parallelopiped, generally constructed of plexiglas for ease of construction and because it is transparent, the parallelopiped having a length L1 of about 20", a height H1 of about 14", and a width W1 of about 12", and having an insulating layer 211 located on the top of the parallelopiped to assist in temperature control. Also, one or more fans (not shown) are generally used to maintain ventilation through chamber 205 for cooling the extraction vessels. A typical material for layer 211 is styrofoam about 1/2" thick. The above dimensions for chamber 205 can vary considerably depending on the desired number and size of extraction vessels, and on the amount of space desired within the chamber to facilitate manipulations of the extraction vessels. Rocker arm 203 is typically a flat sheet of insulating material such plexiglas about 1/4" to 1/2" thick, about 16" long, and about 4" wide. Attached firmly to the rocker arm 203, generally along the length of the rocker arm, is a rocker arm shaft 213, typically metal, which is essentially an extended drive shaft for motor 201. Rocker arm 203 typically has holes bored therethrough to accomodate threaded fittings for holding each of the extraction vessels firmly in place, the extraction vessels typically having a threaded top and the fitting having three holes therethrough to accomodate the teflon lines for gas and liquid flow into the vessels. In the preferred mode, motor 201 is a stepper motor, geared to provide a slow oscillation of approximately one per second of the extraction vessels during mixing operations to avoid shearing of the DNA, that oscillation typically being through an angle of 50° to 60°. During phase separation, the motor 201 is advanced to a position corresponding to full horizontal for the extraction vessels and held for several minutes, and is then returned to normal position (i.e., upright for the extraction vessels), generally over a period of about 10 seconds to ensure that phase separation is maintained. An example of such a motor 201 is an AIRPAX Model K82821-P2 geared down from 0.6 in pitch diameter to 3.6.

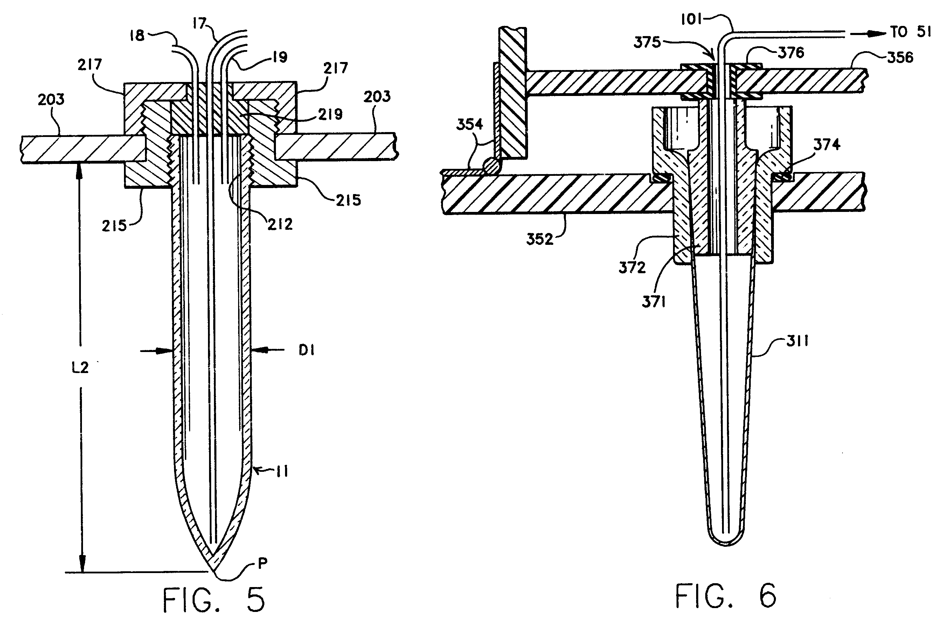

Fig. 5 illustrates a preferred design for the extraction vessels 11 through 15. Each vessel is constructed of glass and has a total length L2 of about 150mm, a maximum outside cross-sectional diameter D1 of about 28mm, and tapers to a point P to facilitate the removal of fluids. In the preferred mode, the vessel is graduated and below a screw top 212 has a volume of about 40ml. An example of such a vessel is a pyrex conical screw cap centrifuge graduated tube available as Corning stock number 8082. The fittings used to hold the vessels in place in the rocker arm 203 are typically constructed of three pieces: a threaded cap 217; a flanged coupling 215 having a inner threaded opening to accept screw top 212, and an outer threaded area to accept cap 217; and a teflon insert 219 which is press fit into flanged coupling 215 and serves as an inert stopper for the extraction vessel. Insert 219 generally has three holes therethrough to accomodate the required liquids and gases. For example, for extracton vessel 11, there is educting tube 17, a pressure tube 18 which is coupled to gas manifold 72, and a vent tube 19 coupled to vent manifold 74. A key feature of this tube is the large change in surface area of the fluid it contains which occurs when the tube is reoriented from the vertical position to a horizontal position, this change in surface area enhancing the rate of phase separation of the emulsion. Other tube shapes can also be used which can attain this change in surface area, and in the general case, the angle of rotation required to increase the surface area of the fluid contained therein may not be 90°.

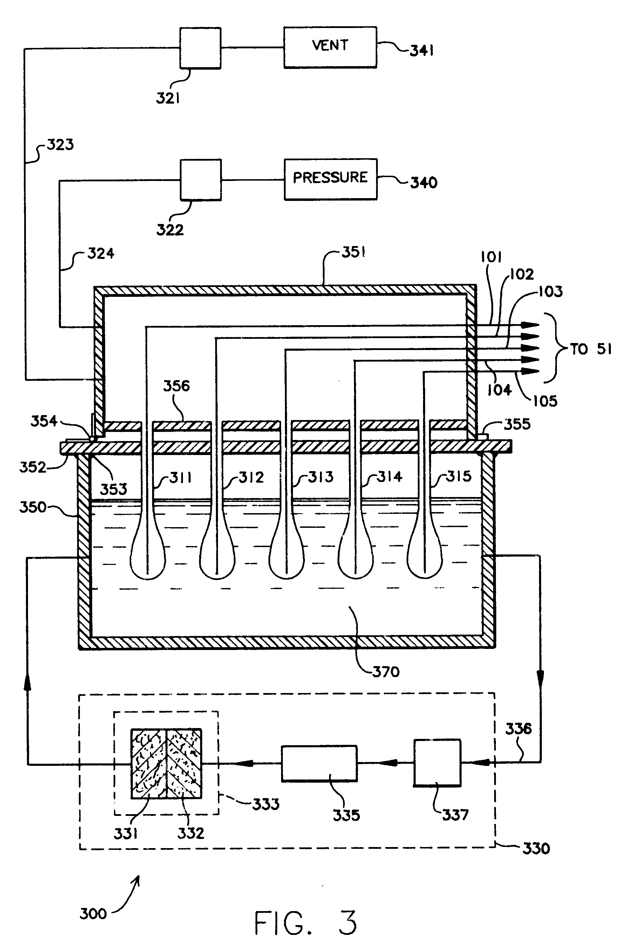

Shown in Fig. 3 is a schematic representation of the pressurized dialysis system 300 which is used for further purifying and concentrating the nucleic acids removed from the extraction vessels 11 through 15. System 300 includes a manifold housing 351 and a bath vessel 350, both of which are typically rectangular in cross-section, the bath vessel generally containing about 8 liters of a dialysate 370. The bath vessel 350 and the manifold housing 351 both abut a cover 352 which is used to exclude foreign matter from the dialysate, with vessel 350 sealed thereto via a seal 353, and manifold housing 351 connected thereto at one side by a hinge 354 and by a clasp 355 at the other side. Bath vessel 350 is generally vented to the ambient atmosphere. In the preferred mode, the bath vessel, the manifold housing and the cover are constructed of plexiglas. Attached to the cover and suspended through holes therein into the dialysate are a plurality of dialysis bags 311 through 315 which are typically in a corresponding relationship with extraction vessels 11 through 15, coupled thereto via valve block 51. Manifold housing 351 includes a bottom piece 356 so that housing 351 is a closed container except for holes through bottom piece 356 to accomodate the dialysis bags, the holes for gas tubes 323 and 324 and for fluid lines 101 through 105 being sealed by feedthroughs. Fig. 6 illustrates the details of the suspension of the dialysis bags and the sealing system so that pressure can be maintained in manifold housing. 351 during dialysis. Generally, the dialysis bags are first attached to a glass fitting independent of the dialysis apparatus. For example dialysis bag 311 is placed over a portion of a short piece of glass tubing 371 which is ground to a taper on one end. Then this tubing 371 and the dialysis bag 311 are pushed firmly into a mated piece of ground glass tubing 372 and given a turn to effect a seal between the two pieces of tubing thereby holding the dialysis bag firmly between them.

The cover 352 is typically drilled to accomodate the larger tubing 372 and the dialysis bag 311 and is countersunk to accomodate an o-ring seal 374. The dialysis bag and the glass fitting made up of tubing 371 and 372 is then placed through the hole. Bottom piece 356 has a hole 375 which is aligned with the glass fitting when the manifold housing is rotated to the closed position about hinge 354. A grommet seal 376 is located in hole 375 and is used to affect a seal between the glass fitting and the manifold.

The dialysis system 300 also includes a recirculation system 330 having a recirculation tube 336 for extracting dialysate 370 from the bath vessel, a peristaltic pump 337 for pumping the dialysate through the recirculation system, a spectrophotometer 335 for monitoring the absorbance of the dialysate, and a dual in-line filter 333 for filtering out phenol and other organic materials. In a typical implementation, filter 333 includes a carbonaceous filter 332 for removing organic materials, and a mixed bed ion-exchange resin filter 331 for removing inorganic material. Also, spectrophotometer 335 typically measures absorbance at 270nm to provide a measure of the phenol remaining in the dialysate. The system generally sets a flag when the absorbance (A270) drops to 0.01 or below, indicating to the computer system that the dialysis function is complete. During dialysis operations, pressure in manifold housing 351 is maintained using an inert gas such as nitrogen and is generally maintained at about 1200mm Hg (guage) when using dialysis bags such as collodion bags from Schleicher and Schuell having a volume of 2 to 8 ml. Pump 337 typically provides a head of 15 psi and a flow rate of about 1l/min. Double distilled water is typically used as dialysate 370. The purpose of this recirculation system is to allow the dialysate to be reused instead of being replenished at regular intervals thereby further facilitating automatic operation.

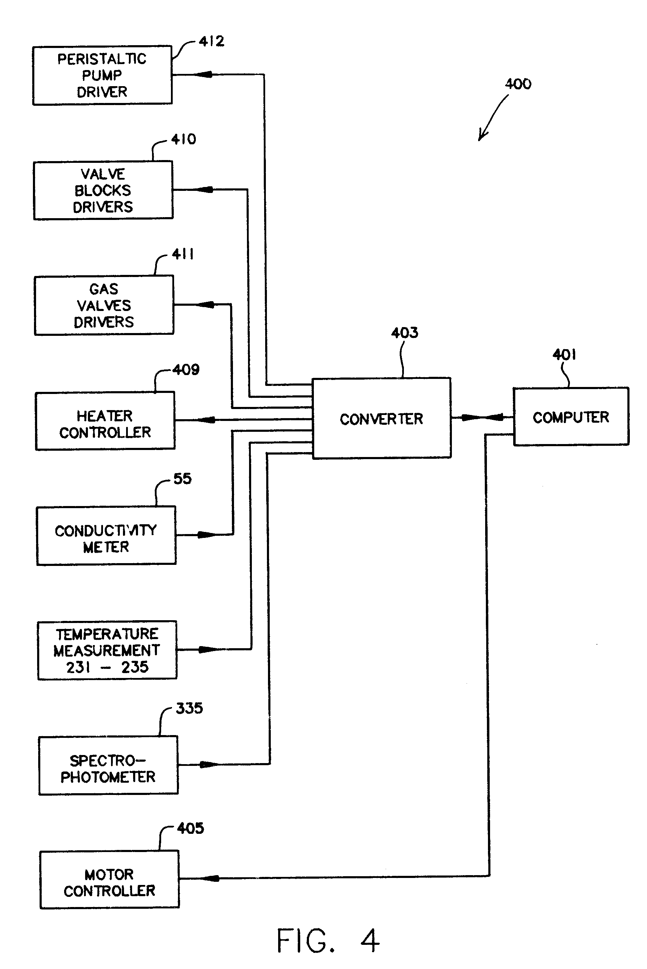

Fig. 4 shows a schematic representation of the computer system 400 used for automatic control. The system is made up of a microprocessor based computer 401 such as a Hewlett-Packard 85, which is coupled to a converter system 403, for converting digital signals from the computer 401 to analog signals to drive a heater controller 409 for controlling the heating of the extraction vessels during extraction, and to provide input signals to drivers 410, 411, and 412 which control the solenoids of the valve blocks, the gas valves, and the peristaltic pump 436, respectively. Converter 403 also serves as an analog to digital converter for providing signals to the computer 401 from spectrophotometer 335 and from conductivity meter 55, and from thermister 231 through 235. An example of such a converter 403 is a Hewlett-Packard 3497 interface. The computer also provides signals to a motor controller 405 for controlling a stepper motor used to oscillate the extraction vessels during mixing operations and to positon the extraction vessels horizontally for phase separation. A typical example of such a controller 405 is a Modulynx™ Motion Control Interface Card, type 10D005A from Superior Electric.

Computer Software System

At the most basic level, software control of the extraction apparatus is a matter of opening and closing valves and turning switches on and off at the proper times to achieve the desired flows of the various materials from one vessel to another and to perform the required operations. The fact that the method of the invention is a sequence of steps lends itself conveniently to software control. The following is an example of a specific instruction set for each step of the extraction method which can be easily translated into whatever programming language it is desired to use. It is based on the assumption that reagent vessel 1 contains the lysis buffer, reagent vessel 2 contains the proteinase K, reagent vessel 3 contains the phenol/chloroform/isoamyl alcohol, reagent vessel 4 contains chloroform, and reagent vessel 5 contains RNase.

STEP 1:   TISSUE DIGESTION

| Command Number | Command |
|---|---|
| 10 | Open valves 22; 50(port 91); 51(port 80, 81); 61. Close all valves 4 minutes after command 10. |
| Comment: | Delivery of lysis buffer to vessel 1 is now complete. |
| 30 | Open valves 24; 50(port 92); 51(ports 80,81); 61. |
| 40 | Close all valves 0.5 minutes after command 30. |
| Comment: | Delivery of proteinase K is now complete. |
| 50 | Turn on heater 221 of vessel 1; raise temperature to 55°C and maintain. |
| 60 | Turn on motor 201; angle of rotation set for $\pm 60°$ with period of 1 second. |
| 70 | Turn off motor 201 and heater 221 3 hours after command 60. |
| 80 | Wait for cooling of digested mixture. |

STEP 2:   EXTRACTION

| | |
|---|---|
| 90 | Open valves 26; 50(port 93); 51(ports 80, 81); 61. |
| 100 | Close all valves except valve 61, 5 minutes after command 90. |

| | | |
|---|---|---|
| Comment: | | Delivery of the extraction mixture (phenol/chloroform/isoamyl alcohol) is now complete. |
| | 110 | Turn on motor 201; angle of rotation set for ±60°; period 1 second. |
| | 120 | Turn off motor 201, 20 minutes after command 110. |
| STEP 3: | SEPARATE PHASES | |
| | 140 | Turn on motor 201, angle of rotation set for 90°. |
| | 150 | Turn off motor 201 when angle of rotation reaches 90°. |
| Comment: | | Extraction vessel is now being held in a horizontal position. |
| | 160 | Turn on thermister 231; increase temperature to 55°C and maintain. |
| | 170 | Turn off thermister 231 12 minutes after temperature reaches 55°C under command 150. |
| STEP 4: | RETURN EXTRACTION VESSSEL TO UPRIGHT POSITON | |
| | 180 | Turn on motor 201, 12 minutes after temperature reaches 55°C; angle of rotation set for 0°; descent rate set for 9°/second. |
| STEP 5: | WITHDRAW PHENOL PHASE | |
| | 190 | Close valve 61. |
| | 200 | Open valves 62; 51(port 81); 52(ports 71, 72). |
| | 210 | Monitor conductivity with conductivity meter 55. |
| | 220 | 1.0 seconds after conductivity reaches $10^4$ Mhos, close all valves. |

| | |
|---|---|
| Comment: | The one second delay in command 220 after the conductivity goes high is to ensure that residual phenol left in the delivery lines to valve block 52 has been removed. |
| 230 | Open valves 52(port 74); 61. |
| 240 | Close all valves 30 seconds after command 230. |
| 250 | Open valves 52(ports 71, 73); 39; 51(port 81); 61. |
| Comment: | Command 250 backflushes valve block 52 and forces aqueous solution left in the delivery lines back into extraction vessel 11 for further extraction or purification. |
| 260 | Close all valves. |

STEP 6:    REPEAT EXTRACTION PROCESS

| | |
|---|---|
| 270 | Perform commands 90 through 250, N times. |
| Comment: | N, the number of extractions performed, is chosen by the programmer based on experience and on the type of sample tissue. |
| 280 | Open valves 28; 50(port 94); 51(port 80, 81); 61. |
| Comment: | Command 280 adds chloroform to extraction vessel 11 for the final extraction. |
| 290 | Close all valves except valve 61. |
| 300 | Repeat steps 110 through 261 one time. |

STEP 7:     REMOVE AQUEOUS SOLUTION (To Dialysis)

310             Open valves 51(port 81, 82); 62; 321.

320             Close all valves 5 minutes after command 310.

Comment:        The aqueous solution in extraction vessel 11 is now in dialysis bag 411.

STEP 8:     DIALYZE AQUEOUS SOLUTION

330             Turn on pump 337.

340             Open valve 324.

350             Monitor A270 with spectrophotometer 335.

360             When A270 is less than or equal to 0.01, close all valves and turn off pump 337.

STEP 9:     REMOVE RNA FROM AQUEOUS SOLUTION (Optional)

370             Open valve 324; open valve 51(ports 81, 82); 61.

380             Close all valves 5 minutes after command 370.

Comment:        The dialyzed aqueous solution in dialysis bag 311 is in extraction vessel 11.

390             Open valves 30; 50(port 95); 51(ports 80, 81); 61.

400             Repeat Steps 90 through 360.

STEP 10:    COLLECT SAMPLE

410             Open valves 324; 51(port 82); 52(ports 71, 75).

420             Close all valves 5 minutes after command 410.

Utility of the Invention

Examples. Preparation of DNA from Human Lymphocytes

Lymphocytes are first washed from one unit of whole blood and are resuspended in 4 ml balanced salt solution. (Balanced salt solution is made up of 1 volume of a solution A and 9 volumes of a solution B, whore solution A is 0.1% glucose, $5 \times 10^{-10}$M $CaCl_2$, $9.8 \times 10^{-4}$M $MgCl_2$, $5.4 \times 10^{-3}$M KCl, 0.145M Tris-HCl, pH 7.6; and Solution B is 0.14M NaCl.) Then 0.35 ml of the lymphocyte suspension above is mixed with 4 ml lysis buffer (1M NaCl, 1% SDS, 8M urea, 10 mM EDTA, 50 mM Tris-HCl, pH 8.0), and 1 mg of proteinase K in 0.65 ml lysis buffer to obtain a total volume of 5 ml. The digestion is performed in a conical tube (extraction vessel 11) as described earlier, at 55°C for 3 hours. About 5 ml of phenol/chloroform/isoamyl alcohol 50:48:2 is added to the tube and the two phases are mixed according to the protocol for 20 minutes. The extraction vessel is then rotated to the horizontal position for 10 minutes at 55°C to allow the phases to separate. The extraction vessel is then rotated slowly back up to the vertical position over a period of about 10 seconds, and the lower organic layer is removed to waste. A second extraction is performed with the phenol/chloroform/isoamyl alcohol mixture and a third extraction is performed using 5 ml of chloroform. The chloroform is removed to waste and the aqueous DNA-containing layer is pressure transferred to a dialysis bag, such as dialysis bag 311. This aqueous solution is then pressure dialyzed according to the protocol until A270 is below 0.01. The final DNA solution is then educted from the bag and collected.

Following this process yields about 1 ml of DNA solution containing about 250 micrograms of DNA The resulting solution had an absorbance ratio A230:A260 of 0.52 and an absorbance ratio A260:A280 of 1.90, demonstrating very high purity. (For absolutely pure DNA, the absorbance ratio A230:A260 is 0.5±0.05 and A260:A280 is 1.9±0.1.) Analysis of the sample using a 0.8% agarose gel and standard ethidium bromide staining techniques shows a single band with a size greater than 50 kbase pairs (i.e., greater than $3.5 \times 10^7$ daltons). Also most importantly, digestion with the enzyme Eco RI is positive, indicating that the DNA is pure and therefore restrictable, a very stringent test for DNA purity.

Variations on the above example demonstrate the importance of heating and increasing the surface area to effect the phase separation step. For example, for human lymphocytes, if the extraction vessel is not heated, but is maintained at room temperature, and the extraction vessel is not rotated to a horizontal position, phase separation typically requires over 60 minutes. If instead the extraction vessel is heated to 55°C but is not also rotated to the horizontal position, the phase separation requires over 4 minutes. With heating to 55°C and rotation of the extraction vessel to the horizontal position, the phase separation typically requires only about 2.5 minutes. In each of these variations, however, the high salt content is enhancing the rate of phase separation by approximately a factor of two.

While there has been shown and described a preferred embodiment of the apparatus and method of the present invention, it will be apparent to those skilled in the art that many changes and modifications may be made without departing from the invention in its broader aspects. For example, it is apparent that the extraction vessel need not be rotated to a horizontal position to speed up phase separation, although it does have a major influence. Similarly, the phase separation can be performed at a temperature below the preferred range of 45°C to 55°C, but it will proceed at a slower rate, and the further below that range the slower the rate. In terms of apparatus it will be apparent that automated devices according to the invention can be constructed with either more or fewer extraction vessels, reagent vessels, and dialysis bags. Also, some valves nay be conveniently placed at different locations in the apparatus, for example valve block 52 may be placed ahead of conductivity meter 55. However, this would result in some loss of the aqueous phase when the flow is stopped. In addition, the specific model numbers chosen for the various pieces of apparatus included in the automated extraction system are not meant to be restrictive as to the particular models which can be used, but are offered by way of example only. Also, it will be apparent that the apparatus may be only partially automated, by providing the fluid delivery system 100 and chamber/rocker system 200 independent of the dialysis system 300.

## Claims

1. A method of extracting nucleic acids from cells which includes the steps of
   (a) creating a lysate by treating said cells with proteinase K in the presence of a lysis buffer;
   (b) mixing said lysate with a phenol-based solvent system, thereby creating an emulsion; and
   (c) separating the phases into an aqueous phase containing said nucleic acids and an organic phase containing phenol and denatured proteins characterised by
       (i) heating said emulsion to a temperature greater than 45°C prior to separation to cause phase separation; and
       (ii) removing said organic phase without centrifugation.

2. A method according to claim 1, wherein the lysis buffer contains a high concentration of salt.

3. A method according to claim 1 or claim 2, wherein the said temperature is about 55°C.

4. A method according to any preceding claim wherein after the heating step but prior to separation, the surface area of the emulsion is increased thereby further promoting phase separation.

5. A method according to any preceding claim wherein the steps of mixing, heating (surface extension if employed) and separating are repeated, in order, N times, where N is a number greater than or equal to 1.

6. A method according to any preceding claim wherein after final separation the remaining aqueous phase is mixed with chloroform thereby creating an emulsion and the heating and separation steps are repeated to leave an aqueous phase containing said nucleic acids.

7. A method according to claim 6 wherein after the steps of mixing, heating, surface extension and separation have been repeated N times, the remaining aqueous phase is mixed with chloroform thereby creating an emulsion, and the steps of heating, surface extension and separation are repeated in that order to leave an aqueous phase containing said nucleic acids.

8. The method of claim 5 wherein the remaining aqueous phase after mixing with chloroform is dialyzed.

9. A method according to any preceding claim wherein said phenol-based solvent system comprises a mixture of phenol, chloroform, and an antisurfactant.

10. The method of claim 8 wherein the ratios of phenol to chloroform to isoamyl alcohol are about 50:48:1.

11. The method of any preceding claim wherein said lysis buffer includes a detergent.

12. The method of claim 10 wherein said lysis buffer inlcudes one or more of a chaotropic agent, a chelating agent, and a buffer.

13. A method according to any preceding claim wherein the molar concentration of salt in said lysis buffer is from 0.5 and 2.

14. A method according to claim 13 wherein said lysis buffer comprises urea, NaCl, a detergent, Tris-HCℓ, and EDTA.

15. The method of claim 14 wherein the lysis buffer comprises 8M urea, 1M NaCl, 1% SDS, 50mM Tris-HCℓ, and 10mM EDTA, pH 8.0.

16. Apparatus for extracting nucleic acids from cells according to the method of claim 1, the apparatus comprising:
    extraction vessel means (11-15) for holding said cells:
    delivery means (100) for delivering reagents to said extraction vessel means, and for removing fluids from said extraction vessel means:
    heater means (221-225) for heating said extraction vessel means;
    temperature measuring means (231-235) for measuring the temperature of said extraction vessel means;
    motor means (200) for oscillating said extraction vessel means to mix said cells and said reagents;
    computer means (400) for controlling said heater means, said motor means and said delivery means, for controlling volume of flow of said reagents into said extraction vessel means, for monitoring the temperature measured by said temperature measuring means, for monitoring flow of fluids from said extraction vessel means, for timing the heating of said extraction vessels by said heating means, and for timing the mixing by said motor means, said computer means operating according to a preselected instruction set to cause extraction of said nucleic acids from said cells without centrifugation.

17. Apparatus according to claim 16 wherein said extraction vessel means is configured such that when it is rotated about a horizontal axis by an angle, a fluid contained therein will have a larger surface area than when said extraction vessel means is not rotated, and wherein said motor means is arranged for rotating said extraction vessel means to said angle and for holding said extraction vessel means at said angle at least for a period to permit phase separations of emulsions contained in said extraction vessel means.

18. Apparatus according to claim 16 or claim 17 wherein said extraction vessel means comprises a plurality of tubes.

19. Apparatus according to any of claims 16 to 18 further comprising dialysis means (300) coupled to said extraction vessel means via said delivery means, and said delivery means is arranged for delivering fluids to and from said dialysis means.

20. Apparatus according to claim 19 wherein said dialysis means comprises recirculation means for recirculating and filtering dyalysate during dialysis.

21. Apparatus according to any one of claims 16 to 20 wherein
the extraction vessel (11-15) has a cross-section area A1 in a horizontal plane through the extraction vessel, and has a cross-sectional area A2 in a plane oriented at an angle $\theta$ relative to the horizontal, where A2 is greater than A1; and the
motor means has a driveshaft coupled to the extraction vessel for rotating the extraction vessel by angle $\theta$, and for holding the extraction vessel at angle $\theta$ for a period during or prior to the phase separation.

22. Apparatus according to claim 21 comprising also means for withdrawing the lower phase and control means for the withdrawing means, the control means including means for detecting a change in conductivity.

23. Apparatus according to any one of claims 16 to 22 comprising also:
holding means for holding reagents to be reacted;
one or more extraction vessels (11-15);
first valve block means having a plurality of ports and being responsive to signals from the computer means for directing delivery of the reagents to the extraction vessel(s);
delivery means for delivering the reagents to the first valve block means from the holding means, and to the extraction vessel(s), and for removing fluids from bottom portion(s) of the extraction vessel-(s), and for delivering the fluids from the bottom portion(s) to the first valve block means; and
measuring means coupled to said first valve block means for receiving fluids therefrom and for measuring conductivity of the fluids received from the first valve block means, and providing a signal to the computer means when the conductivity is above a preselected threshold.

**Patentansprüche**

1. Verfahren zum Extrahieren von Nukleinsäuren aus Zellen, welches die Stufen aufweist:
(a) Erzeugen eines Lysats durch Behandlung der Zellen mit Proteinase K in der Anwesenheit eines Lysispuffers;
(b) Mischen des Lysats mit einem auf Phenol basierenden Lösemittelsystem, um auf diese Art und Weise eine Emulsion zu erzeugen; und
(c) Separieren der Phasen in eine wässrige Phase, die die Nukleinsäuren enthält, und eine organische Phase, die das Phenol und die denaturierten Proteine enthält, gekennzeichnet durch
(i) Erhitzen der Emulsion auf eine Temperatur höher als 45 °C vor der Trennung, um eine Phasenseparation zu erzeugen; und
(ii) Entfernen der organischen Phase ohne zu zentrifugieren.

2. Verfahren nach Anspruch 1, wobei der Lysispuffer eine hohe Konzentration an Salz enthält.

3. Verfahren nach Anspruch 1 oder Anspruch 2, wobei die Temperatur um 55 °C liegt.

4. Verfahren nach irgendeinem der vorangehenden Ansprüche, wobei nach der Erhitzungsstufe, jedoch vor der Separation, der Oberflächenbereich der Emulsion erhöht wird, um auf diese Art und Weise die Phasenseparation zu fördern.

5. Verfahren nach irgendeinem der vorangehenden Ansprüche, wobei die Stufen des Mischens, des Erhitzens (Oberflächenentspannung, falls angezeigt) und des Trennens N-mal wiederholt werden, wobei N eine Zahl größer oder gleich 1 ist.

6. Verfahren nach irgendeinem der vorangehenden Ansprüche, wobei nach der endgültigen Separation die verbleibende wässrige Phase mit Chloroform vermischt wird, um auf diese Art und Weise eine Emulsion zu erzeugen, und wobei die Erhitzungs- und die Separationsstufen wiederholt werden, um eine wässrige Phase zurückzubehalten, die die Nukleinsäuren enthält.

7. Verfahren nach Anspruch 6, wobei, nachdem die Stufen des Mischens, des Erhitzens, der Oberflächenentspannung und der Separation N-mal wiederholt worden sind, die verbleibende wässrige Phase mit Chloroform vermischt wird, um auf diese Art und Weise eine Emulsion zu erzeugen, und wobei die Stufen der Erhitzung, der Oberflächenspannung und der Separation so oft wiederholt werden, um eine wässrige Phase zurückzubehalten, die die Nukleinsäuren enthält.

8. Verfahren nach Anspruch 5, wobei die verbleibende wässrige Phase nach dem Mischen mit Chloroform einer Dialyse unterzogen wird.

9. Verfahren nach irgendeinem der vorangehenden Ansprüche, wobei das auf Phenol basierende Lösemittelsystem eine Mischung aus Phenol, Chloroform und einem oberflächeninaktiven Mittel aufweist.

10. Verfahren nach Anspruch 8, wobei das Verhältnis von Phenol zu Chloroform zu Isoamylalkohol ungefähr bei 50:48:1 liegt.

11. Verfahren nach irgendeinem der vorangehenden Ansprüche, wobei der Lysispuffer ein Detergenz enthält.

12. Verfahren nach Anspruch 10, wobei der Lysispuffer einen oder mehrere chaotropische Agenzien, ein Gelieragenz und einen Puffer enthält.

13. Verfahren nach irgendeinem der vorangehenden Ansprüche, wobei die molare Konzentration des Salzes in dem Lysispuffer zwischen 0,5 und 2 liegt.

14. Verfahren nach Anspruch 13, wobei der Lysispuffer Harnstoff, NaCl, ein Detergenz, Tris-HCl und EDTA aufweist.

15. Verfahren nach Anspruch 14, wobei der Lysispuffer 8M Harnstoff, 1M NaCl, 1% SDS, 50mM Tris-HCl und 10mM EDTA mit dem PH-Wert 8,0 aufweist.

16. Vorrichtung zum Extrahieren von Nukleinsäuren aus Zellen nach dem Verfahrensanspruch, wobei die Vorrichtung aufweist:
Extraktionsgefäßeinrichtungen (11 - 15) zum Aufbewahren der Zellen:
Ausgabevorrichtungen (100) zur Ausgabe von Reagenzien in die Extraktionsgefäßeinrichtung und zum Entfernen von Flüssigkeiten aus der Extraktionsgefäßeinrichtung:
Heizeinrichtungen (221 - 225) zum Erhitzen der Extraktionsgefäßeinrichtung;
Temperaturmeßeinrichtungen (231 - 235) zum Messen der Temperatur der Extraktionsgefäßeinrichtung;
Motoreinrichtungen (200) zum Inschwingungsetzen der Extraktionsgefäßeinrichtung, um die Zellen mit den Reagenzien zu vermischen;
Rechnereinrichtungen (400) zur Steuerung der Heizeinrichtungen, der Motoreinrichtungen und der Ausgabevorrichtung, um den Volumenstrom der Reagenzien in die Extraktionsgefäßeinrichtungen hinein zu steuern, um die durch die Temperaturmeßeinrichtungen ermittelten Temperaturen aufzuzeichnen, um den Strom der Flüssigkeiten aus den Extraktionsgefäßeinrichtungen aufzuzeichnen, um die Heizung der Extraktionsgefäßeinrichtungen durch die Heizeinrichtungen zeitlich zu steuern und um die Mischung durch die Motoreinrichtungen zeitlich zu steuern, wobei die Rechnereinrichtungen gemäß einem ausgewählten Instruktionssatz arbeiten, um die Extraktion der Nukleinsäuren aus den Zellen ohne Zentrifuge zu bewirken.

**17.** Vorrichtung nach Anspruch 16, wobei die Extraktionsgefäßeinrichtungen so gestaltet sind, daß, wenn sie um eine horizontale Achse um einen Winkel gedreht werden, eine in ihnen enthaltende Flüssigkeit oder Fluid einen größeren Oberflächenbereich einnimmt, als wenn die Extraktionsgefäßeinrichtungen nicht gedreht werden und wobei die Motoreinrichtungen so angeordnet sind, um die Extraktionsgefäßeinrichtungen um den Winkel zu drehen und, um die Extraktionsgefäßeinrichtungen in diesem Winkel wenigstens für eine Zeitperiode zu halten, um die Phasenseparation der Emulsionen zu ermöglichen, die in den Extraktionsgefäßeinrichtungen enthalten sind.

**18.** Vorrichtung nach Anspruch 16 oder Anspruch 17, worin die Extraktionsgefäßeinrichtungen eine Anzahl von Rohren oder Röhrchen aufweisen.

**19.** Vorrichtung nach irgendeinem der Ansprüche 16 bis 18, weiterhin aufweisend eine Dialyseeinrichtung (300), die an die Extraktionsgefäßeinrichtungen über die Ausgabevorrichtungen angeschlossen ist, und daß die Ausgabevorrichtungen zur Lieferung der Fluide und Flüssigkeiten in die Dialyseeinrichtung und aus dieser heraus eingerichtet sind.

**20.** Vorrichtung nach Anspruch 19, worin die Dialyseeinrichtung eine Rezirkulationseinrichtung zur Rezirkulation und Filterung des Dialysats während der Dialyse aufweist.

**21.** Vorrichtung nach irgendeinem der Ansprüche 16 bis 20, worin
das Extraktionsgefäß (11 - 15) einen Querschnittsbereich A1 in einer horizontalen Ebene durch das Extraktionsgefäß und einen Querschnittsbereich A2 in einer Ebene in einem Winkel $\theta$ relativ zur Horizontalen hat, wobei A2 größer als A1 ist; und
die Motoreinrichtungen eine Antriebswelle aufweisen, die mit dem Extraktionsgefäß zwecks Drehung des Extraktionsgefäßes um den Winkel $\theta$ und zwecks Halterung des Extraktionsgefäßes in einem Winkel $\theta$ über eine Zeitspanne während oder vor der Separationsphase verbunden ist.

**22.** Vorrichtung nach Anspruch 21, aufweisend eine Einrichtung zum Zurückziehen der unteren Phase und eine Steuereinrichtung für diese Einrichtung, wobei die Steuereinrichtung eine Einrichtung zum Feststellen einer Veränderung in der Leitfähigkeit aufweist.

**23.** Vorrichtung nach einem der Ansprüche 16 bis 22, aufweisend
eine Halteeinrichtung zum Aufbewahren der miteinander reagierenden Reagenzien;
eine oder mehrere Extraktionsgefäße (11 - 15);
einen ersten Ventilblock mit einer Mehrzahl von Einlässen, der auf Signale von der Rechnereinrichtung zur Steuerung der Ausgabe der Reagenzien zu den Extraktionsgefäßen oder dem Extraktionsgefäß anspricht;
eine Ausgabeeinrichtung zur Lieferung der Reagenzien in den ersten Ventilblock von der Aufbewahrungseinrichtung und zu dem oder den Extraktionsgefäßen und zum Entfernen der Flüssigkeiten aus dem Bodenteil oder den Bodenteilen des oder der Extraktionsgefäße und zur Ausgabe der Flüssigkeiten von dem oder den Bodenbereichen zu dem ersten Ventilblock; und
Meßeinrichtungen, die mit dem ersten Ventilblock verbunden sind, um von dorther die Flüssigkeiten zu empfangen und die Leitfähigkeit der Flüssigkeiten zu messen, die von dem ersten Ventilblock erhalten worden sind und um ein Signal zu der Rechnereinrichtung zu geben, wenn die Leitfähigkeit oberhalb einer vorgegebenen Schwelle liegt.

**Revendications**

**1.** Procédé pour l'extraction d'acides nucléiques à partir de cellules, comprenant les étapes consistant à
(a) créer un lysat en traitant lesdites cellules avec de la protéinase K en présence d'un tampon de lyse ;
(b) mélanger ledit lysat avec un système de solvant à base de phénol, pour ainsi créer une émulsion ; et
(c) séparer les phases en une phase aqueuse contenant lesdits acides nucléiques et une phase organique contenant du phénol et des protéines dénaturées, caractérisé par
(i) le chauffage de ladite émulsion à une température supérieure à 45° C avant la séparation pour provoquer ladite séparation des phases ; et
(ii) l'élimination de ladite phase organique sans centrifugation.

**2.** Procédé selon la revendication 1, dans lequel le tampon de lyse contient une forte concentration en sel.

**3.** Procédé selon la revendication 1 ou la revendication 2, dans lequel ladite température est d'environ 55° C.

**4.** Procédé selon l'une quelconque des revendications précédentes, dans lequel, après l'étape de chauffage mais avant la séparation, la superficie de l'émulsion est augmentée pour ainsi favoriser davantage la séparation des phases.

**5.** Procédé selon l'une quelconque des revendications précédentes, dans lequel les étapes de mélange, chauffage (augmentation de la surface, le cas échéant) et de séparation sont répétées, dans l'ordre, N fois, N étant un nombre supérieur ou égal à 1.

**6.** Procédé selon l'une quelconque des revendications précédentes, dans lequel, après la séparation finale, la phase aqueuse restante est mélangée à du chloroforme pour ainsi créer une émulsion, et les étapes de chauffage et de séparation sont répétées pour laisser une phase aqueuse contenant lesdits acides nucléiques.

**7.** Procédé selon la revendication 6, dans lequel, après que les étapes de mélange, de chauffage, d'augmentation de la surface et de séparation ont été répétées N fois, la phase aqueuse restante est mélangée à du chloroforme pour ainsi créer une émulsion, et les étapes de chauffage, d'augmentation de la surface et de séparation sont répétées dans cet ordre pour laisser une phase aqueuse contenant lesdits acides nucléiques.

**8.** Procédé selon la revendication 5, dans lequel la phase aqueuse restante, après le mélange avec le chloroforme, est dialysée.

**9.** Procédé selon l'une quelconque des revendications précédentes, dans lequel ledit système de solvant à base de phénol comprend un mélange de phénol, de chloroforme et un antisurfactant.

**10.** Procédé selon la revendication 8, dans lequel les rapports entre le phénol, le chloroforme et l'alcool isoamylique sont d'environ 50:48:1.

**11.** Procédé selon l'une quelconque des revendications précédentes, dans lequel ledit tampon de lyse contient un détergent.

**12.** Procédé selon la revendication 10, dans lequel ledit tampon de lyse contient un ou plusieurs parmi un agent chaotropique, un agent chélatant et un tampon.

**13.** Procédé selon l'une quelconque des revendications précédentes, dans lequel la concentration molaire de sel dans ledit tampon de lyse est de 0,5 à 2.

**14.** Procédé selon la revendication 13, dans lequel ledit tampon de lyse comprend de l'urée, du NaCl, un détergent, du Tris-HCl et de l'EDTA.

**15.** Procédé selon la revendication 14, dans lequel le tampon de lyse comprend 8 M d'urée, 1 M de NaCl, 1 % de SDS, 50 mM de Tris-HCl, et 10 mM d'EDTA, pH 8,0.

**16.** Appareil pour l'extraction d'acides nucléiques à partir de cellules, selon le procédé de la revendication 1, l'appareil comprenant :

des moyens formant vase(s) d'extraction (11 - 15) pour contenir lesdites cellules ;

des moyens d'amenée (100) pour amener des réactifs auxdits moyens formant vases d'extraction et pour retirer des fluides desdits moyens formant vases d'extraction ;

des moyens chauffants (221 - 225) pour chauffer lesdits moyens formant vases d'extraction ;

des moyens de mesure de température (231 - 235) pour mesurer la température desdits moyens formant vases d'extraction ;

des moyens formant moteur (200) pour faire osciller lesdits moyens formant vases d'extraction pour mélanger lesdites cellules et lesdits réactifs;

des moyens formant ordinateur (400) pour commander lesdits moyens chauffants, lesdits moyens formant moteur et lesdits moyens d'amenée, pour commander le volume d'écoulement desdits réactifs dans lesdits moyens formant vases d'extraction, pour surveiller la température mesurée par lesdits moyens de mesure de température, pour surveiller l'écoulement de fluides à partir desdits moyens formant vases d'extraction, pour cadencer le chauffage desdits vases d'extraction par lesdits moyens chauffants et pour cadencer le mélange par lesdits moyens formant moteur, lesdits moyens formant ordinateur fonctionnant selon un jeu d'instructions présélectionné pour provoquer l'extraction desdits acides nucléiques à partir desdites cellules sans centrifugation.

17. Appareil selon la revendication 16, dans lequel lesdits moyens formant vases d'extraction sont configurés de telle manière que lorsqu'ils tournent d'un angle autour d'un axe horizontal, un fluide contenu à l'intérieur aura une plus grande superficie que lorsque lesdits moyens formant vases d'extraction ne sont pas tournés, et dans lequel lesdits moyens formant moteur sont propres à faire tourner lesdits moyens formant vases d'extraction dudit angle et à maintenir lesdits moyens formant vases d'extraction audit angle au moins pendant une période permettant les séparations des phases d'émulsions contenues dans lesdits moyens formant vases d'extraction.

18. Appareil selon la revendication 16 ou la revendication 17, dans lequel lesdits moyens formant vases d'extraction comprennent plusieurs tubes.

19. Appareil selon l'une quelconque des revendications 16 à 18, comprenant en outre des moyens de dialyse (300) reliés auxdits moyens formant vases d'extraction par l'intermédiaire desdits moyens d'amenée, et dans lequel lesdits moyens d'amenée sont propres à acheminer des fluides vers lesdits moyens de dialyse et à partir de ceux-ci.

20. Appareil selon la revendication 19, dans lequel lesdits moyens de dialyse comprennent des moyens de recirculation pour faire recirculer et filtrer le dialysat pendant la dialyse.

21. Appareil selon l'une quelconque des revendications 16 à 20, dans lequel

le vase d'extraction (11 - 15) a une aire de la section transversale A1 dans un plan horizontal traversant le vase d'extraction et a une aire de la section transversale A2 dans un plan orienté selon un angle θ par rapport à l'horizontale, A2 étant supérieure à A1 ; et

les moyens formant moteur ont un arbre de commande couplé au vase d'extraction pour faire tourner le vase d'extraction de l'angle θ et pour maintenir le vase d'extraction à l'angle θ pendant une période au cours de la phase de séparation ou avant celle-ci.

22. Appareil selon la revendication 21, comprenant des moyens pour prélever la phase inférieure et des moyens de commande pour les moyens de prélèvement, les moyens de commande comprenant des moyens pour détecter une variation de conductivité.

23. Appareil selon l'une quelconque des revendications 16 à 22, comprenant en outre

des moyens formant récipients pour contenir des réactifs destinés à être mis à réagir ;

un ou plusieurs vases d'extraction (11 - 15) ;

des premiers moyens formant bloc de soupapes ayant une pluralité d'orifices et réagissant à des signaux venant des moyens formant ordinateur pour diriger l'acheminement des réactifs vers le ou les vases d'extraction ;

des moyens d'amenée pour acheminer les réactifs vers les premiers moyens formant bloc de soupapes à partir des moyens formant récipients et vers le ou les vases d'extraction, et pour évacuer les fluides depuis la ou les parties de fond du ou des vases d'extraction, et pour acheminer les fluides depuis la ou les parties de fond vers les premiers moyens formant bloc de soupapes ; et

des moyens de mesure reliés auxdits premiers moyens formant bloc de soupapes pour recevoir de ceux-ci des fluides et pour mesurer la conductivité des fluides reçus à partir des premiers moyens formant bloc de soupapes et pour fournir un signal aux moyens formant ordinateur lorsque la conductivité est supérieure à un seuil présélectionné.

FIG. 1

EP 0 215 533 B1

FIG. 2a

FIG. 2b

FIG. 3

FIG. 4

FIG. 5

FIG. 6

EP 0 215 533 B1